# NEUE EUROPÄISCHE PATENTSCHRIFT

(11) **EP 1 140 139 B2**
(45) Veröffentlichungstag und Bekanntmachung des Hinweises auf die Entscheidung über den Einspruch: **18.03.2009**
(45) Hinweis auf die Patenterteilung: 12.11.2003
(21) Anmeldenummer: 99966958.3
(22) Anmeldetag: 13.12.1999
(51) Int. Cl.: A61K 38/18, A61P 9/10

(54) **VERWENDUNG VON ERYTHROPOIETIN ODER ERYTHROPOIETIN-DERIVATEN ZUR BEHANDLUNG VON CEREBRALEN ISCHÄMIEN**
USE OF ERYTHROPOIETIN OR ERYTHROPOIETIN DERIVATIVES FOR THE TREATMENT OF CEREBRAL ISCHAEMIA
UTILISATION DE L'ERYTHROPOIETINE OU DE DERIVES DE L'ERYTHROPOIETINE POUR TRAITER LES ISCHEMIES CEREBRALES

(30) Priorität: 14.12.1998 DE 19857609
(43) Veröffentlichungstag der Anmeldung: 10.10.2001
(73) Patentinhaber: Ehrenreich, Hannelore, 37077 Göttingen (DE); Gleiter, Christoph, 72074 Tübingen (DE)
(72) Erfinder: Ehrenreich, Hannelore, 37077 Göttingen (DE); Gleiter, Christoph, 72074 Tübingen (DE)
(74) Vertreter: Pfenning, Meinig & Partner GbR
(86) Internationale Anmeldenummer: PCT/EP1999/009864
(87) Internationale Veröffentlichungsnummer: WO 2000/035475

(56) Entgegenhaltungen:
- WO-A-97/14307
- RUSCHER K ET AL: "Erythropoietin protects neurons from oxygen/glucose deprivation induced cell death." SOCIETY FOR NEUROSCIENCE ABSTRACTS, Bd. 24, Nr. 1-2, 1998, Seite 298 XP000923282 28th Annual Meeting of the Society for Neuroscience, Part 1;Los Angeles, California, USA; November 7-12, 1998 ISSN: 0190-5295
- SAKANAKA M ET AL : "In vivo evidence that erythropoietin protects neurons from ischemic damage" PROC NATL ACAD SCI USA, Bd. 95, April 1998 (1998-04), Seiten 4635-4640, XP002142085 in der Anmeldung erwähnt
- BUNDESVERBAND DER PHARMAZEUTISCHEN INDUSTRIE E.V.: "Rote Liste 1998" 1998 , AULENDORF/WURTT., EDITION CANTOR.; DE XP002142086 NeoRecormon-Fachinfo-Service 08045
- PATENT ABSTRACTS OF JAPAN vol. 018, no. 001 (C-1148), 6. Januar 1994 (1994-01-06) & JP 05 246885 A (CHUGAI PHARMACEUT CO LTD), 24. September 1993 (1993-09-24)
- DEL MASTRO L ET AL: "Strategies for the use of epoetin alfa in breast cancer patients" ONCOLOGIST, Bd. 3, Nr. 5, 1998, Seiten 314-318, XP000923283 in der Anmeldung erwähnt
- MARTI H H ET AL: "Protective role of erythropoietin in brain ischemia." PHYSIOLOGICAL RESEARCH, Bd. 48, Nr. SUPPL. 1, 1999, Seite S14 XP000923284 Second Congress of the Federation of European Physiological Societies and Czech Physiological Society;Prague, Czech Republic; June 30-July 4, 1999 ISSN: 0862-8408
- ALBAYRAK S. ET AL: 'Effect of transient focal ischemia on blood-brain barrier permeability in the rat : correlation to cell inury' ACTA NEUROPATHOL Bd. 94, 1997, Seiten 158 - 163
- MARTI H.H. ET AL: 'Detection of erypoeitin in human liquor : Intrinsic erythropoietin production in the brain' KIDNEY INTERNATIONAL Bd. 51, 1997, Seiten 146 - 418
- S. A. MENZIES ET AL.: 'Extravasation of albumin in ischaemic brain oedema' ACTA NEUROCHIRURGICA Bd. SUPPL. 51, 1990, Seiten 220 - 222
- R. SUZUKI ET AL.: 'The effect of 5-minute ischemia in mongolian gerbils: I. Blood-brain barrier, cerebral glucose flow, and local cerebral glucose utilization changes' ACTA NEUROPATHOL. Bd. 60, 1983, Seiten 207 - 216
- E. M. LOBERG ET AL.: 'Neuronal uptake of plasma proteins after transient cerebral ischemia/hypoxia' APMIS Bd. 101, 1993, Seiten 777 - 783

## Beschreibung

Die vorliegende Erfindung bezieht sich auf ein Mittel zur Behandlung von Schlaganfall des Menschen, wie sie beispielsweise bei Schlaganfallpatienten auftreten.

Beim ischämischen Hirninfarkt unterscheidet man die geschädigten Bereiche in ischämische Kernzone sowie die den Kern umgebende sogenannte Penumbra. Das Ausmaß von ischämischem Kern plus Penumbra determiniert die Schadensgröße nach ischämischem Insult.

Erythropoietin, auch abgekürzt als "EPO" bezeichnet, ist ein körpereigenes Glycoprotein mit einem Molekulargewicht von 30000 Dalton (W. Jelkmann, "Erythropoietin: Structure, Control of Production, and Function", Physiological Reviews, 1992, Band 72, Seiten 449 bis 489). Es ist ein wesentlicher Wachstumsfaktor für die Produktion von Erythrozyten und wurde bereits 1977 erstmals isoliert.

Erythropoietin ist seit vielen Jahren in häufigem klinischem Gebrauch bei Patienten mit renaler Anämie bei Nephrodialyse, zur Gewinnung größerer Mengen autologen Blutes vor geplanten Operationen und geriet auch als Dopingmittel in die Schlagzeilen der Presse.

Dabei erwies sich Erythropoietin als ausgesprochen gut verträglich. Als relevante Nebenwirkungen sind insbesondere die oft therapeutisch erwünschte Stimulation der Hämatopoiese mit Polyglobulie sowie eine selten zu beobachtende arterielle Hypertonie zu nennen. Beide Auswirkungen sind hauptsächlich nach chronischer Erythropoietin-Gabe zu erwarten. Sie sind im Bedarfsfall relativ einfach durch medikamentöse Behandlung bzw. Aderlaß abzuhelfen. Unverträglichkeitsreaktionen bzw. anaphylaktische Reaktionen gehören bei Erythropoietin zu den Raritäten.

Bis heute gibt es keine wirksame Therapie cerebraler Ischämien, wie beispielsweise zur Behandlung von Schlaganfallpatienten, ohne Eingriff in den Kopfbereich des Patienten.

Sakanaka M. et al. offenbaren in PNAS 1998, Band 95, nr. 8, S. 4635 - 4640, daß die zentrale Gabe von Erythropoietin im Tierversuch eine protektive Wirkung auf cerebrale Neuronen ausübt. Aufgrund der Kenntnis, daß die Blut-Hirn-Schranke durch größere Proteine nicht überwunden werden kann, erfolgt in sämtlichen Versuchen die Gabe des Erythropoietin direkt zentral in den lateralen Ventrikel. Eine derartige unmittelbar intraventrikuläre Gabe des Erythropoietin, d.h. unmittelbare Infusion des Erythropoietin in das Hirngewebe scheidet jedoch beim Menschen aufgrund der hohen Risiken, die mit der Anlage und dem Unterhalt einer temporären Ventrikeldrainage verbunden sind, beispielsweise Infektionen oder Blutungen, aus.

DelMastro L. et al. offenbaren in Oncologist 1998, 3/5, S. 314-318, daß die präventive Gabe von Erythropoietin eine Anämie in chemotherapeutisch behandelten Krebspatienten verhindern und damit präventiv das Risiko derartiger Patienten bzgl. einer cerebralen Ischämie in Folge einer chemotherapeutisch verursachten Anämie reduzieren kann. Eine Therapie für eine bereits vorliegende cerebrale Ischämie, insbesondere bei nicht chemotherapeutisch behandelten Patienten, ist darin nicht offenbart.

Aufgabe der vorliegenden Erfindung ist es daher, ein Verwendung zur Herstellung von Arzneimitteln zur Behandlung von Schlaganfall zur Verfügung zu stellen, die einfach und möglichst nebenwirkungsfrei sowie risikenfrei angewandt werden können.

Diese Aufgabe wird durch die Verwendung zur Herstellung eines Arzneimittels nach Anspruch 1 gelöst. Vorteilhafte Weiterbildungen der erfindungsgemäßen Verwendung werden in den abhängigen Ansprüchen gegeben.

Ansatzpunkt für die erfindungsgemäßen Verwendungen von Erythropoietin ist es, daß nach stattgefundener Ischämie nach einem Schlaganfall, möglichst früh so viel wie möglich des geschädigten Hirngewebes, insbesondere der Penumbra, gerettet werden sollte. Es wurde gefunden, daß eine periphere Gabe von Erythropoietin einen ausgeprägt protektiven Effekt auf das von der Ischämie betroffene cerebrale Gewebe aufweist. Erythropoietin bewirkt dabei, daß der Bereich des geschädigten cerebralen Gewebes, insbesondere in der Penumbra, dramatisch verkeinert wird gegenüber herkömmlichen Maßnahmen bei cerebraler Ischämie ohne Erythropoietingabe.

Diese unerwartete gewebsrettende Wirkung des peripher gegebenen Erythropoietins bei cerebraler Ischämie des Menschen nach einem Schlaganfall ist schon deshalb nicht selbstverständlich, da Erythropoietin bekanntermaßen als größeres Protein mit einem Molekulargewicht von ca. 30000 Dalton die Blut-Hirn-Schranke gewöhnlich nicht zu überwinden vermag. Eine unmittelbar intraventrikuläre Gabe des Erythropoietin, d.h. unmittelbare Infusion des Erythropoietin in das Hirngewebe scheidet jedoch beim Menschen gewöhnlich aus wegen Risiken, die mit der Anlage und dem Unterhalt einer temporären Ventrikeldrainage verbunden sind, wie Infektionen oder Blutungen.

Es ist das Verdienst der vorliegenden Erfindung, zu erkennen und umzusetzen, daß überraschenderweise zur Behandlung einer stattgehabten cerebralen Ischämie nach einem Schlaganfall unmittelbar nach dem schädigenden Ereignis Erythropoietin peripher als Arzneimittel gegeben werden kann und dann in das geschädigte Hirnareal übergeht und wirksam wird.

Die periphere Gabe von Erythropoietin, d.h. diesseits der Blut-Hirn-Schranke, erfolgt vorteilhafterweise intramuskulär oder vaskulär. Eine unmittelbar vaskuläre Gabe, die bekanntermaßen vorteilhafterweise bei Arzneimitteln meist intravenös erfolgen sollte, bietet sich hier geradezu an, um das Erythropoietin innerhalb eines kurzen Zeitraums, d.h. so rasch wie möglich nach dem schädigenden Ereignis, in einer hohen Dosis zu dem geschädigten cerebralen Gewebe zu bringen.

Es ist daher davon auszugehen, daß Erythropoietin unmittelbar nach der Schädigung des Hirngewebes durch die Ischämie in den geschädigten Bereichen die Blut-Hirn-Schranke überwinden kann. Daher ist es möglich, dem durch einen Schlaganfall geschädigten Patienten ein Arzneimittel, das Erythropoietin enthält, zu verabreichen, wobei das Erythropoietin tatsächlich das geschädigte Hirngewebe erreicht.

Damit steht erstmals ein wirksames Therapeutikum für die cerebrale Ischämie beim Menschen bei einem Schlaganfall, zur Verfügung.

Vorteilhaft ist dabei weiterhin, daß die intakte Blut-Hirn-Schranke in den nicht-geschädigten cerebralen Gewebebereichen ein Eindringen des dort nicht benötigten Erythropoietin weiterhin wirksam verhindert und so die nicht von dem ischämischen Infarkt betroffenen Gewebebereiche von der Therapie nicht beeinflußt werden, also auch keine oder nur stark verringerte Nebenwirkungen auftreten können.

Vorteilhafterweise wird Erythropoietin als Arzneimittel mit einer Dosierung in Höhe von 5000 bis 100000 Einheiten, idealerweise von 35000 Einheiten, pro Gabe, möglichst mit einer Gabe pro Tag in den ersten Tagen, erstmals möglichst innerhalb von 8 Stunden nach dem Schlaganfall, angewandt. Es genügen dabei einige wenige Gaben von Erythropoietin, um den therapeutischen Effekt herbeizuführen. Dies hat weiterhin den Vorteil, daß die hauptsächlich bei langdauernden, kontinuierlichen Behandlungen anderer Krankheitsbilder nach dem oben beschriebenen Stand der Technik beobachteten Nebenwirkungen und Risiken bei der Verwendung von Erythropoietin zur Behandlung cerebraler Ischämie nicht oder nur geringfügig auftreten werden.

Erythropoietin ist aus dem Stand der Technik bekannt. Auch weitere Varianten des Erythropoietins mit veränderter Aminosäuresequenz oder -struktur oder auch Bruchstücke mit den für die biologische Funktion des Erythropoietin relevanten funktionellen Sequenzabschnitten können für die erfindungsgemäße Verwendung eingesetzt werden. Variabilität ergibt sich weiterhin aus der Glycosilierung des Erythropoietins.

Im folgenden werden Beispiele für die erfindungsgemäßen Verwendung gegeben.

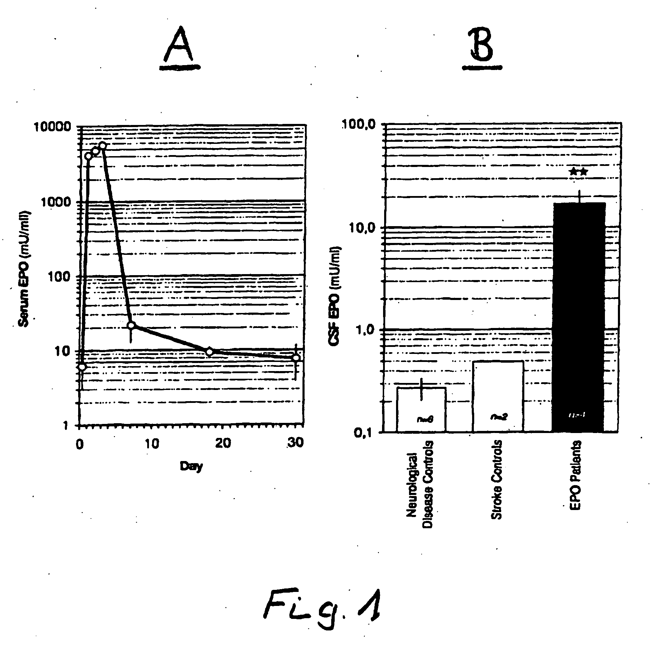

Es zeigen: Fig 1 das Auftreten von Erythropoietin im Serum und in der Cerebrospinalflüssigkeit nach einem Schlaganfall, sowie

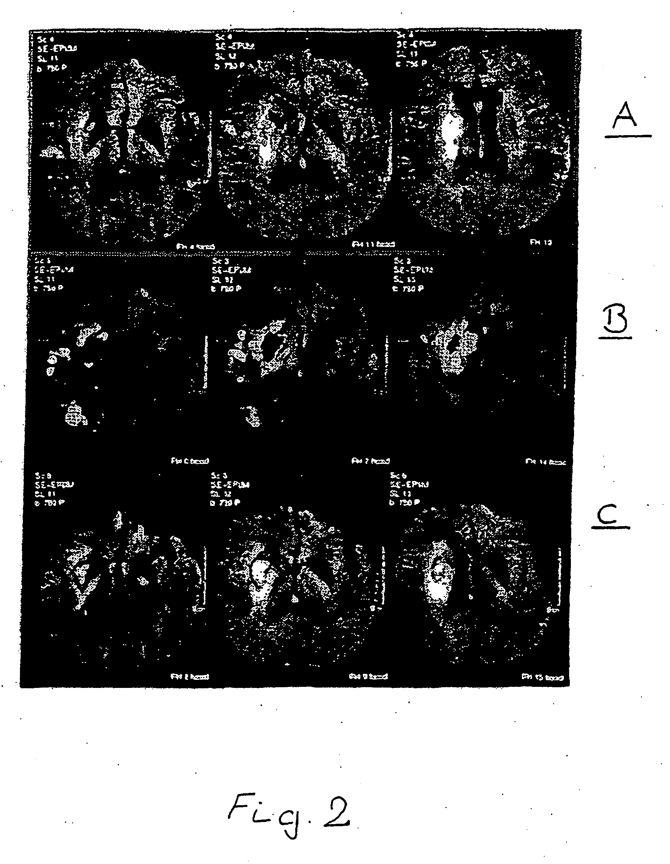

Fig. 2 das Läsionsausmaß nach cerebraler Ischämie.

In Fig. 1A ist der Mittelwert der Serumkonzentration von 4 Patienten mit Schlaganfall, d.h. deren periphere Konzentration von Erythropoietin über mehrere Tage aufgetragen, denen ca. 8 Stunden, ca. 24 Stunden und nochmals ca. 48 Stunden nach dem Schlaganfall jeweils eine Dosis von 35000 IE humanes rekombinantes Erythropoietin (Präparat "Neorecormon" der Fa. Hoffmann-LaRoche AG) intravenös gegeben wurde. Es ist zu erkennen, daß die Serumkonzentration innerhalb der ersten Tage ihr Maximum erreicht und anschließend wieder stark abfällt.

In Fig. 1B sind die Konzentrationen von EPO in sechs Kontrollpatienten mit nichtischämischen neurologischen Erkrankungen ("Neurological Desease Controls") nach Infusion von Erythropoietin, in zwei unbehandelten Schlaganfallspatienten ("Stroke Controls") ohne Infusion von Erythropoietin sowie in vier Schlaganfallspatienten ("EPO-Patients") nach Infusion von Erythropoietin wie bei den Kontrollpatienten dargestellt. Dargestellt ist dabei der Mittelwert der EPO-Konzentration in der Cerebrospinalflüssigkeit, wie er im Mittel 6,4 Stunden nach einer ersten Infusion von 35000 IE humanen rekombinanten Erythropoietins (Präparat "Neorecormon" der Fa. Hoffmann-LaRoche AG) bestimmt wurde. Bei den vier Schlaganfallpatienten ("EPO-Patients") handelt es sich um dieselben Patienten wie in Fig. 1A.

Es ist, unter Berücksichtigung der verwendeten logarithmischen Skala der Darstellung in Fig. 1B, unmittelbar zu erkennen, daß die Konzentration des Erythropoietin in der Cerebrospinalflüssigkeit in Schlaganfallspatienten ("EPO-Patients") um das ca. 100-fache über der der gleich behandelten Kontrollpatienten ("Neurological Disease Controls") oder auch der unbehandelten Schlaganfallspatienten ("Stroke-Controls") liegt.

Es ist das Verdienst der vorliegenden Erfindung, zu erkennen, daß im Falle einer cerebralen Ischämie die Blut-Hirn-Schranke für Erythropoietin durchlässig wird, so daß zur Behandlung einer cerebralen Ischämie nach einem Schlaganfall unmittelbar nach dem schädigenden Ereignis Erythropoietin peripher als Arzneimittel in das geschädigte Hirnareal übergehen und wirksam werden kann.

Fig. 2 zeigt das Läsionsausmaß nach einem Schlaganfall bei einem 73 Jahre alten Patienten. Die gezeigten Aufnahmen wurden mittels magnetischer Kernresonanzspektroskopie ("diffusion-weighted MRI") erzeugt.

Der Patient wurde ca. 8 Stunden nach einem Schlaganfall mit 35000 IE humanem rekombinantem Erythropoietin (Präparat "Neorecormon" der Fa. Hoffmann-LaRoche AG) intravenös infundiert. Ca. 24 Stunden und 48 Stunden nach dem Schlaganfall wurde jeweils eine weitere gleich hohe Dosis Erythropoietin gegeben.

Fig 2A zeigt dabei drei Schnittansichten von unten während des Therapieverlaufs durch das Gehirn des Patienten ca. 7 Stunden nach dem Schlaganfall. Deutlich durch ihre weiße Färbung abgesetzt sind die durch den Schlaganfall geschädigten Bereiche zu erkennen.

In Fig. 2B sind die geschädigten Bereiche ca. 3 Tage nach dem Schlaganfall ebenfalls durch ihre weißliche Färbung (mit dunklem Kern) zu erkennen..

Fig. 2C zeigt dieselben Schnittansichten nach 18 Tagen. Deutlich zu erkennen ist, daß es zu einer markanten Reduktion der Primärläsion kam. Diese Reduktion des ischämischen Infarktareals ist u.a. der Behandlung mit Erythropoietin zuzuschreiben.

## Patentansprüche

1. Verwendung von Erythropoietin zur Herstellung eines peripher zu applizierenden Arzneimittels zur Behandlung von Schlaganfall beim Menschen..

2. Verwendung nach dem vorhergehenden Anspruch zur Herstellung eines vaskulär zu applizierenden Arzneimittels.

3. Verwendung nach dem vorhergehenden Anspruch zur Herstellung eines intravenös zu applizierenden Arzneimittels.

4. Verwendung nach einem der Ansprüche 1 bis 3 in einer Dosis von 5000 IE bis 100000 IE pro Gabe und/oder pro Tag.

5. Verwendung nach einem der Ansprüche 1 bis 4 in einer Dosis von 35000 IE pro Gabe und/oder pro Tag.

6. Verwendung nach einem der Ansprüche 1 bis 5, **dadurch gekennzeichnet, daß** als Erythropoietin ein natives oder rekombinantes menschliches oder tierisches Erythropoietin oder eine Variante hiervon verwendet wird.

## Claims

1. The use of erythropoietin for the manufacture of a medicinal product to be applied peripherally for the treatment of stroke in humans.

2. Use according to the above claim for the manufacture of a medicinal product to be applied vascularly.

3. Use according to the above claim for the manufacture of a medicinal product to be applied intravenously.

4. Use according to one of claims 1 to 3 in a dose of 5,000 IE to 100,000 IE per administration and/or per day.

5. Use of one of claims 1 to 4 in a dose of 35,000 IE per administration and/or per day.

6. Use according to one of the claims 1 to 5 **characterised in that** a native or recombinant human or animal erythropoietin or a variant of this is used as erythropoietin.

## Revendications

1. Utilisation d'érythropoïétine pour la fabrication d'un médicament à administrer par voie périphérique pour le traitement d'une attaque cérébrale chez l'être humain.

2. Utilisation selon la revendication précédente pour la fabrication d'un médicament à administrer par voie vasculaire.

3. Utilisation selon l'une des revendications précédentes pour la fabrication d'un médicament à administrer par voie intraveineuse.

4. Utilisation selon l'une des revendications 1 à 3 à une dose de 5 000 unités antitoxiques (IE) à 100 000 unités antitoxiques (IE) par prise et/ou par jour.

5. Utilisation selon l'une des revendications 1 à 4 à une dose de 35 000 unités antitoxiques (IE) par prise et/ou par jour.

6. Utilisation selon l'une des revendications 1 à 5, **caractérisée en ce que** l'érythropoïétine utilisée est une érythropoïétine naturelle ou une érythropoïétine recombinante humaine ou animale, ou une variante de ces dernières.
